# EUROPEAN PATENT APPLICATION

(11) **EP 4 800 110 A1**
(43) Date of publication of application: **02.09.2026**
(21) Application number: 25161001.0
(22) Date of filing: 28.02.2025
(51) Int. Cl.: C12N 15/113

(54) **GAPMER ANTISENSE OLIGONUCLEOTIDE FOR TARGETING THE RHO GENE**

(71) Applicant: Eberhard Karls Universität Tübingen (Medizinische Fakultät), 72074 Tübingen (DE)
(72) Inventor: De Angeli, Pietro, 72076 Tübingen (DE); Wissinger, Bernd, 72127 Kusterdingen (DE)
(74) Representative: Witte, Weller & Partner Patentanwälte mbB

(57) **Abstract**

The present invention relates to a gapmer antisense oligonucleotide (gAON) for targeting a RHO gene, a pharmaceutical composition for the treatment of retinitis pigmentosa (RP) comprising said gAON, a method for modulating the expression of the human RHO gene involving said gAON, and to a method for the prophylaxis and/or treatment of a living being against and/or suffering from RP involving said gAON.

## Description

### FIELD OF THE INVENTION

The present invention relates to a gapmer antisense oligonucleotide (gAON) for targeting a *RHO* gene, a pharmaceutical composition for the treatment of retinitis pigmentosa (RP) comprising said gAON, a method for modulating the expression of the human *RHO* gene involving said gAON, and to a method for the prophylaxis and/or treatment of a living being against and/or suffering from RP involving said gAON.

### BACKGROUND OF THE INVENTION

Retinitis pigmentosa (RP) is a member of a group of genetic disorders called inherited retinal dystrophy (IRD) that cause loss of vision. Retinitis pigmentosa is the most prevalent form of inherited retinal disease, affecting approx. 1 in 5000 people worldwide. RP is generally inherited from one or both parents. Within the spectrum of RP, autosomal dominant RP (adRP) accounts for approx. 25-30% of all RP cases.

The underlying mechanism involves the progressive loss of rod photoreceptor cells that line the retina of the eyeball. The rod cells secrete a neuroprotective substance (rod-derived cone viability factor, RdCVF) that protects the cone cells from apoptosis. When these rod cells die, this substance is no longer provided. This is generally followed by the loss of cone photoreceptor cells.

There is currently no cure for retinitis pigmentosa. Efforts to manage the problem may include the use of low vision aids, portable lighting, or orientation and mobility training. The efficiency of various supplements, such as vitamin A, DHA, NAC, and lutein, in delaying disease progression remains an unresolved, yet prospective treatment option. A visual prosthesis may be an option for people with severe symptoms.

Retinitis pigmentosa is caused by genetic variants in nearly 100 genes. Mutations in the *RHO* gene encoding rhodopsin represent the most common causative factor, mostly acting as gain-of-function or dominant-negative mutations. Because of these underlying pathological effects, gene supplementation gene therapy is not a viable therapeutic option. Consequently, main research efforts towards gene-based therapies for *RHO*-associated adRP have focused on "knockout and replace strategies", which combine the knockout or knockdown of the endogenous *RHO* gene, both the mutant and the wildtype allele, with the simultaneous delivery of a knockout/knockdown-resistant wildtype *RHO* cDNA.

However, while promising pre-clinical results have been obtained in animal models, a major unsolved hurdle still remains, which is controlling the expression level of the RHO transgene in transduced cells. There is only one FDA-approved gene therapy that is commercially available to RP patients with Leber congenital amaurosis type 2. It replaces the miscoded RPE65 protein that is produced within the retinal pigmented epithelium. It has been found to be effective in approximately 50% of the patients who receive the therapy.

The current therapeutic approaches for the treatment of rhodopsin-mediated autosomal dominant Retinitis Pigmentosa are summarized in the following review article: Meng et al. (2020), Therapy in Rhodopsin-Mediated Autosomal Dominant Retinitis Pigmentosa, Mol. Ther. 28(10):2139-2149.

Murrey et al. (2015), Allele-Specific Inhibition of Rhodopsin With an Antisense Oligonucleotide Slows Photoreceptor Cell Degeneration, Invest. Ophthalmol. Vis. Sci. 56(11): 6362-75, describe in an animal experiment on rats that the intravirteale administration of an antisense oligonucleotide (ASO) in a form of retinitis pigmentosa caused by a mutated P23H-rhodopsin gene led to an improvement in photoreceptor function and thickening of the outer nuclear layer in the eye. The authors claim that they were able to slow down the degeneration process of the photoreceptors by using an ASO and thus reducing the amount of mutant P23H rhodopsin.

WO 2020/219983 describes chemically modified oligonucleotides that are proposed for the treatment and prevention of RHO-related diseases, such as retinitis pigmentosa. In particular, such oligonucleotides are described that are directed against P23H-mutated RHO.

WO 2023/285431 discloses a CRISPR/Cas-based method proposed for the treatment of retinitis pigmentosa. Specifically, guide RNA molecules are provided that target the single nucleotide polymorphism rs7984 located in the 5' untranslated region (UTR) of the *RHO* gene.

Even as little as 23% of RHO overexpression is retinotoxic in mice and copy number variants in wildtype *RHO* have now been reported as a novel cause of adRP. In contrast, reduced *RHO* gene dosage does not result in disease, as evidenced by early nonsense mutations, exhibiting a recessive inheritance pattern.

In addition, more than 200 different missense mutations in the *RHO* gene are now known to cause adRP. Due to this highly heterogeneous mutational spectrum, beside knock-out/down and replace strategies requiring multiple components and influencing the finely-tuned endogenous expression of the gene, therapeutic strategies able to address multiple mutations have not yet been developed.

### SUMMARY OF THE INVENTION

It is therefore one of the objects underlying the invention to provide a new strategy for achieving improved treatment and prophylaxis of a RP that is based on a mutation in the *RHO* gene. In particular, a substance is to be provided that is suitable as an active agent of a pharmaceutical composition for the treatment and prophylaxis of RP.

The object underlying the invention is achieved by a gapmer antisense oligonucleotide (gAON) that specifically binds to and/or is complementary to a polynucleotide comprising the nucleotide sequence of SEQ ID NO: 1 and/or SEQ ID NO: 2.

The inventors have succeeded in developing a substance that can be used to selectively reduce the translation of any mutated *RHO,* i.e. without having to restrict it to a specific mutation. The invention utilizes a benign single nucleotide polymorphism that is frequently present in heterozygosity, i.e. in about > 30% of the general population. The gapmer antisense oligonucleotide (gAON) according to the invention is designed to specifically target and silence the c.-26A or c.-26G allele that lies in *cis* with any *RHO* mutation. Retinitis pigmentosa (RP) is mostly autosomal dominant, meaning that one *RHO* allele is mutated, and the other *RHO* allele is wild-type. The single nucleotide polymorphism means that in RP patients, one allele would mostly be c.-26A and the other allele would mostly be c.-26G. The phase of the mutant allele, c.-26A or c.-26G, can be readily determined in a RP patient. With this information, the RP patient can be treated with the A- or G-directed gAON of the invention, depending on which of the two alleles the RP patient's *RHO* mutation is located.

According to the invention, the target sequence of the gAON on the mutated *RHO*-encoding molecule, e.g. the mRNA and/or pre-mRNA, is, at its minimum, TGGGTGGGAGCAGCC(A/G)CGGGTCAGCCACAAG (SEQ ID NO: 1) on the DNA level, and UGGGUGGGAGCAGCC(A/G)CGGGUCAGCCACAAG (SEQ ID NO: 2) on the RNA level. A/G can also be named as "R".

According to the invention, a "gapmer antisense oligonucleotide (gAON)" is a specific form of an antisense oligonucleotide. It consists of at least one short, mostly synthetically produced single-stranded DNA sequence ("gap") and modified flanks ("wings"). The central gap region is usually 8-10 nucleotides long and typically consists of unmodified deoxyribonucleic acid (DNA). It enables binding to the complementary pre-mRNA or mRNA sequence via Watson-Crick base pairing and serves as a substrate for the enzyme RNase or RNase H. The ends or flanks of the gapmer consist of about 2-5 mostly chemically modified nucleotides (e.g. locked nucleic acids (LNA), 2'-*O*-methyl-ribonucleotides or 2'-methoxyethoxy (M*O*E)). These modifications improve stability against nucleolytic degradation, increase affinity for the target RNA, and reduce potential off-target effects. Modifications are also often made to the phosphodiester backbone, such as phosphorothioate bonds, which further optimize stability and pharmacokinetics. The total length of a gAON is therefore typically about 15-20 nucleotides. The gapmer binds specifically to a complementary pre-mRNA or mRNA sequence by base pairing. The central DNA gap enables the binding of RNase H, a cellular enzyme that degrades RNA in RNA-DNA hybrids. After binding, RNase H selectively degrades the target mRNA, e.g. the pre-mRNA or mRNA of the mutated *RHO,* thus preventing the translation of the encoded protein. This ultimately leads to a reduction in the expression of the target gene, i.e. the mutated RHO at the protein level.

According to the invention, "specific binding" means a selective and preferential hybridization of the gAON with a complementary target nucleic acid sequence, e.g. mRNA or pre-mRNA via Watson-Crick base pairing under stringent conditions (e.g. defined salt concentration, temperature and pH) that allow the formation of stable nucleic acid hybrids. The gAON has a low dissociation constant (K_d) for the target sequence, indicating a strong and stable binding. Typically, the T_m of the gAON-target hybrid should be at least 5-10°C higher than that of hybrids with non-targeted sequences of the same length and similar nucleotide composition. Binding of a gAON to its target can easily be assessed by the person skilled in the art using techniques that are known in the AON field in general, such as the gel mobility shift assay as, e.g., described in Hellman, L. M., and Fried, M. G. (2007). "Electrophoretic mobility shift assay (EMSA) for detecting protein-nucleic acid interactions", Nature Protocols, 2(8), 1849-1861.

"Complementary" in the context of the invention refers to the specific and unique sequence match between the gAON and its target nucleic acid molecule (e.g. mRNA, pre-mRNA encoding mutated RHO), which enables stable hybridization through Watson-Crick base pairing. The term "complementary" used in the context of the invention indicates that some mismatches in the antisense sequence are allowed as long as the functionality, i.e. recruiting of RNase, in particular RNase H, is achieved. Preferably, the complementarity is from 90% to 100%. In general, this allows for 1 or 2 mismatches in a gAON of up to 20 nucleotides. Whether or not complementarity exists can be readily determined by methods known to those skilled in the art, e.g. the gel mobility shift assay mentioned above.

The object underlying the invention is fully achieved.

In an embodiment of the invention said gAON is configured to mediate degradation by RNase, in particular RNase H, of an mRNA and/or pre-mRNA encoding the human *RHO* gene, preferably the mutated human *RHO* gene, or fragments thereof.

This measure has the advantage that such a gAON molecule is used that recruits RNase H after binding to the mRNA of the *RHO* gene. This enzyme selectively cleaves the RNA in the RNA-DNA hybrid, leading to the degradation of the target mRNA. The targeted recognition of the mutant mRNA minimizes the degradation of the wild-type mRNA, which reduces potential side effects. Since both complete mRNA and fragments are targeted, the gAON is robust against splice variants and partial transcripts.

In the context of this invention, a "fragment" of an mRNA or pre-mRNA refers to any partial sequence of a complete mRNA or pre-mRNA that comprises sufficient nucleotides to enable specific binding to the gAON and thereby achieve a functional effect, such as recruiting RNase H or influencing the splicing process. A fragment can range from approx. 6 to several hundred nucleotides depending on the specific application. Sequences of approx. 10 to approx. 50 nucleotides are particularly preferred, since these typically allow the binding of a gAON.

In another embodiment of the invention the gAON comprises a DNA sequence ("gap") of approx. 6 to approx. 20 nucleotides, preferably approx. 8 to approx. 15 nucleotides, particularly preferably approx. 10 nucleotides.

The "gap" region is the central DNA segment of a gAON that enables RNase H recruitment. The specified length of approximately 6 to 20 nucleotides provides flexibility by allowing the gAON to adapt to different target sequences of the mRNA or pre-mRNA of the *RHO* gene. On the other hand, it offers optimization of RNase activity, because studies have shown that a gap length of approx. 8 to approx. 10 nucleotides is particularly effective in achieving strong RNase H binding and mRNA degradation. A length of approx. 8 to approx. 15 nucleotides offers a good compromise between specificity and stability. Shorter gaps could promote non-specific binding to non-targeted RNAs. Longer gaps could impair binding affinity and cause structural problems. Approx. 10 nucleotides are particularly preferred. This corresponds to the ideal substrate for RNase H, which maximizes the efficiency of gene silencing.

In still another embodiment of the invention the gap comprises a nucleotide sequence selected from the group consisting of SEQ ID NO: 33 - 36.

The inventors have developed four specific and selective gap regions that lead to particularly favorable results with the gAON. By defining the gap sequence to a selection of specific nucleotide sequences (SEQ ID NO: 33 - 36), the specificity of the gAON is significantly increased. This minimizes off-target effects and ensures that primarily the disease-relevant target RNA is recognized and degraded.

In a further embodiment of the invention the gAON comprises an additional RNA sequence ("wing"), at its 3' and/or 5' terminus, of approx. 2 to approx. 10 nucleotides, preferably approx. 2 to approx. 5 nucleotides, particularly preferably approx. 3 nucleotides. Further, in an embodiment of the invention, the wing comprises modified bases.

The RNA sequence ("wing") at the 3' and/or 5' termini of the gAON provides additional protection against nucleolytic degradation by exonuclease enzymes. Particularly in the extracellular environment or in cell culture medium, where free nucleases are present, the wing contributes to the longer half-life of the gAON. The modified wings support the hybridization of the gAON with the target mRNA or pre-mRNA, especially in the area in front of the coding sequence of the RHO gene. The preferred length of about 3 nucleotides offers a good compromise between strong binding affinity and reduced off-target binding. The precise base spacing provided by the RNA wings optimally exposes the target mRNA, allowing RNase H to efficiently initiate RNA degradation. Chemical modifications can easily be added to the wings. Such modifications not only increase stability, but can also enhance target affinity and reduce unwanted immune responses.

In still another embodiment of the invention the modification of the modified bases in the gAON is selected from the group consisting of 2'-*O*-methylation (2'-M*O*E), locked nucleic acid (LNA), and 2' fluoro (2'-F) modification.

2'-*O*-Methylation (2'-M*O*E) refers to a chemical modification at the 2'-carbon atom of the ribose sugar by attaching a methoxyethoxy group (-OCH₂CH₂OCH₃). Locked nucleic acid (LNA) are modified nucleotides in which the 2'-O-position of the ribose is connected to the 4'-C-position by a methylene bridge. In the case of a 2' fluoro (2'-F) modification, the 2'-hydroxy group (-OH) of the ribose sugar is substituted by a fluorine atom (F).

Chemical modifications such as 2'-*O*-methylation (2'-M*O*E), locked nucleic acid (LNA) and 2' fluoro (2'-F) confer increased stability of the gAON molecule against nucleases that occur naturally in biological systems. This is particularly important for therapeutic applications as it extends the half-life of the gAON in biological liquids and in tissues, thus increasing the duration of action. LNA modifications support base pairing by conformationally fixing the nucleic acid, resulting in stronger and more specific hybridization with the target mRNA. A higher affinity to the target sequence advantageously allows the gAON to be effective at low concentrations, reducing the risk of side effects. Modifications such as 2'-MOE and 2'-F reduce the likelihood of the gAON being recognized by the immune system, particularly by toll-like receptors (TLR) that often respond to single-stranded RNA (ssRNA). A lower immune response means that the risk of inflammatory reactions in the treatment of retinitis pigmentosa is minimized in an advantageous way. The modified wings protect the gAON from degradation, while the gap region continues to allow RNase H recruitment. This ensures that the gAON can efficiently degrade the mutant *RHO* mRNA without losing its structural properties. Because the chemical modifications improve the physical and chemical properties of the gAON, it can also be used in challenging therapeutic scenarios, such as intravitreal application, as required in the treatment of retinal diseases.

In another embodiment the gAON according to the invention comprises the following consensus nucleotide sequence:

X1-X2-X3-X4-X5-X6-X7-X8-X9-G-X10-G-G-C-T-G-X11-X12-X13-X14-X15-X16-X17-X18-X19, with
X1 = absent or G
X2 = absent or G
X3 = absent of C
X4 = absent or T/U
X5 = absent or G
X6 = absent or A
X7 = absent or C
X8 = absent or C
X9 = absent or C
X10 = C or T/U
X11 = absent or C
X12 = absent or T/U
X13 = absent or C
X14 = absent or C
X15 = absent or C
X16 = absent or A
X17 = absent or C
X18 = absent or C
X19 = absent or C

(SEQ ID NO: 37).

The use of a consensus sequence makes it possible to realize a large number of possible nucleotide combinations in the gap region of the gAON. The consensus sequence contains both fixed nucleotides and variable positions (X₁-X₁₉), which can be either specific bases or absent. This variability allows the gAON to be designed to bind specifically to conserved sequences of *RHO* mRNA. This also creates the conditions for personalized therapy, as patient-specific differences can be taken into account. The option of using no base ("absent") at certain positions could help to prevent undesired binding to non-targeted mRNA sequences. This further increases the specificity of the gAON, which improves safety and tolerability in a therapeutic application.

In still another embodiment of the invention the gAON comprises a nucleotide sequence selected from the group consisting of SEQ ID NO: 3 - 32, preferably SEQ ID NO: 3 - 6.

With this measure, the nucleotide sequences developed by the inventors are used, whereby gAON molecules with the sequences SEQ ID NO: 3-6 lead to particularly good results. The nucleotide sequences specified in the SEQ ID NOs are chosen so that they represent optimal target structures for the gAON to bind to the mutated *RHO* mRNA. The gap region of the gAON remains ideal for recruiting RNase H through these specific sequences, which ensures the targeted degradation of the mRNA.

Another subject-matter of the invention relates to a pharmaceutical composition comprising the gAON according to the invention and a pharmaceutically acceptable excipient, preferably the pharmaceutical composition is configured for intravitreal administration.

The delivery of the gAON in a pharmaceutical composition allows for a controlled and safe administration of the therapeutic molecule. Intravitreal injection is a direct method of administration into the vitreous body of the eye, which is particularly effective for eye diseases such as retinitis pigmentosa (RP). Since *RHO* mutations particularly affect the retina, intravitreal injection ensures delivery of the gAON to the rod cells of the retina. Due to the local application, the gAON remains largely in the eye, reducing systemic exposure and associated adverse effects. The intravitreal route of administration allows the development of a sustained release effect, whereby the gAON is released over a longer period of time, minimizing the number of injections required.

A pharmaceutically acceptable excipient is a pharmacologically inactive substance used in drug formulations to aid in the stability, bioavailability and administration of the active agent (here gAON). Examples are buffers, tonicity adjusters, stabilizers, emulsifiers, preservatives or gelling agents. Suitable buffers include phosphate buffered saline (PBS), histidine buffer, acetate buffer, etc. Tonicity adjusters ensure the isotonicity of the solution to avoid osmotic stress in the eye tissue. They include sodium chloride (NaCl), mannitol, glycerol, etc. Suitable stabilizers are e.g. EDTA or trehalose. Further components may include hyaluronic acid or hydroxypropylmethylcellulose (HPMC), both of which are viscosity modulators that increase the viscosity of the solution to prolong the retention time in the vitreous humor and control the release rate of the gAON. Possible preservatives include benza-Ikonium chloride or thiomersal. Antioxidants or humectants are also used. Reference is made to "Handbook of Pharmaceutical Excipients", Rowe et al., 9th edition (2020) or "Remington: The Science and Practice of Pharmacy", 23rd edition (2020), where the skilled person learns about all potential and suitable pharmaceutically acceptable excipients.

A preferred route of administration is through intravitreal injection of an aqueous solution or specially adapted formulation for intraocular administration. EP2425814 discloses an oil in water emulsion especially adapted for intraocular (intravitreal) administration of nucleic acid drugs. This emulsion is less dense than the vitreous fluid, so that the emulsion floats on top of the vitreous, avoiding that the injected drug impairs vision. This emulsion is also suitable for the formulation of the pharmaceutical composition of the invention.

The features, characteristics and advantages disclosed for the gAON of the invention apply likewise to the pharmaceutical composition according to the invention.

Another subject-matter of the invention is the gAON and/or the pharmaceutical composition according to the invention for use as a medicament, preferably for use in the treatment of Retinitis Pigmentosa (RP), preferably autosomal dominant RP.

The features, characteristics and advantages disclosed for the gAON of the invention apply likewise to this subject-matter according to the invention.

Still another subject-matter of the invention is a method for modulating the expression of the human *RHO* gene, preferably the mutated human *RHO* gene, in a biological cell, preferably a rod cell, said method comprising contacting said biological cell with the gAON and/or the pharmaceutical composition according to the invention.

Rod cells are responsible for vision in low light and are the first to be damaged in retinitis pigmentosa. By specifically modulating rod cells, the therapy can be targeted to the main cell type affected, which increases the effectiveness of the treatment.

The features, characteristics and advantages disclosed for the gAON of the invention apply likewise to this method according to the invention.

Another subject-matter of the invention is a method for the prophylaxis and/or treatment of a living being against and/or suffering from RP, preferably autosomal dominant RP, comprising the administration to said living being of the gAON and/or the pharmaceutical composition of the invention.

"Treatment" generally refers to all measures that aim to alleviate the symptoms of a disease, to cure the disease or to slow its progression. In the context of the invention, this means treating Retinitis Pigmentosa by degrading the mutant *RHO* mRNA to prevent the production of the harmful protein and thus to preserve retinal function.

"Prophylaxis" generally means the prevention of a disease by taking measures before clinical symptoms appear. In the context of the invention, prophylaxis means that in at-risk individuals carrying a known *RHO* mutation, the gAON can be used preventively to avoid rod cell damage before the disease develops.

The "living being" covers any being expressing rhodopsin, such as vertebrata, including mammal, e.g. humans, dogs, cats, mice, cattle, primates, etc. It also includes invertebrates, e.g. insects, molluscs, crustaceans, worms etc.

The features, characteristics and advantages disclosed for the gAON of the invention apply likewise to this method according to the invention.

Dose ranges of the gAON or composition according to the invention are preferably designed on the basis of rising dose studies in clinical trials (*in vivo* use) for which rigorous protocol requirements exist. A gAON according to the invention may be used at a dose which ranges from 0.01 and 20 mg/kg, preferably from 0.05 and 20 mg/kg. A suitable intravitreal dose would be between 0.05 mg and 5mg, preferably between 0.1 and 1 mg per eye, such as about per eye: 0.1, 0.2, 0.3, 0.4, 0.5, 0.6, 0.7, 0.8, 0.9 or 1.0 mg.

In a preferred embodiment, a concentration of a gAON as defined herein, which is ranged from 0.1 nM and 1 mM is used. Preferably, this range is for *in vitro* use in a cellular model such as retina cells or retinal tissue. More preferably, the concentration used is ranged from 1 to 400 nM, even more preferably from 10 to 200 nM, even more preferably from 50 to 100 nM. If multiple distinct gAONs are used, this concentration or dose may refer to the total concentration or dose of the gAONs or the concentration or the dose of each gAON added.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (e.g. about 10) preferably means that the value may be the given value (of 10) more or less 5% of the value.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

Reference is made to the following figures:

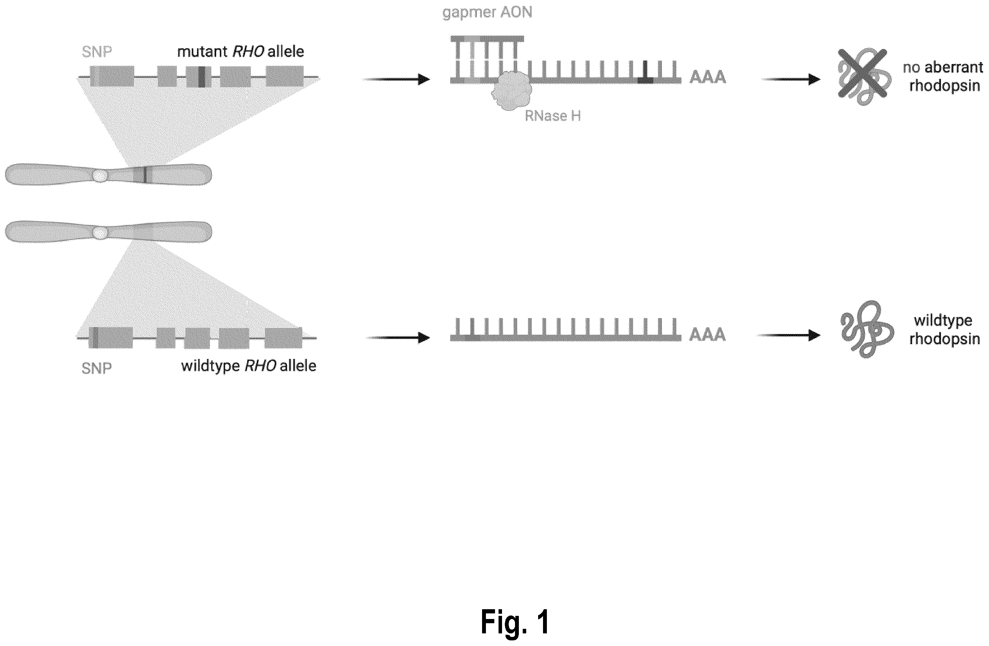

- Fig. 1:: Strategy of allele-specific, mutation-independent AON approach for knock-down of mutant *RHO* alleles. Instead of targeting the individual pathogenic variants (red), the gapmer AONs are designed to hybridize to a common single nucleotide polymorphism (SNP) in the *RHO* mRNA. If the patient is heterozygous for the SNP, only the disease-causing *RHO* allele will be degraded by an RNase H - dependent mechanism, leading to reduced expression of mutant protein.

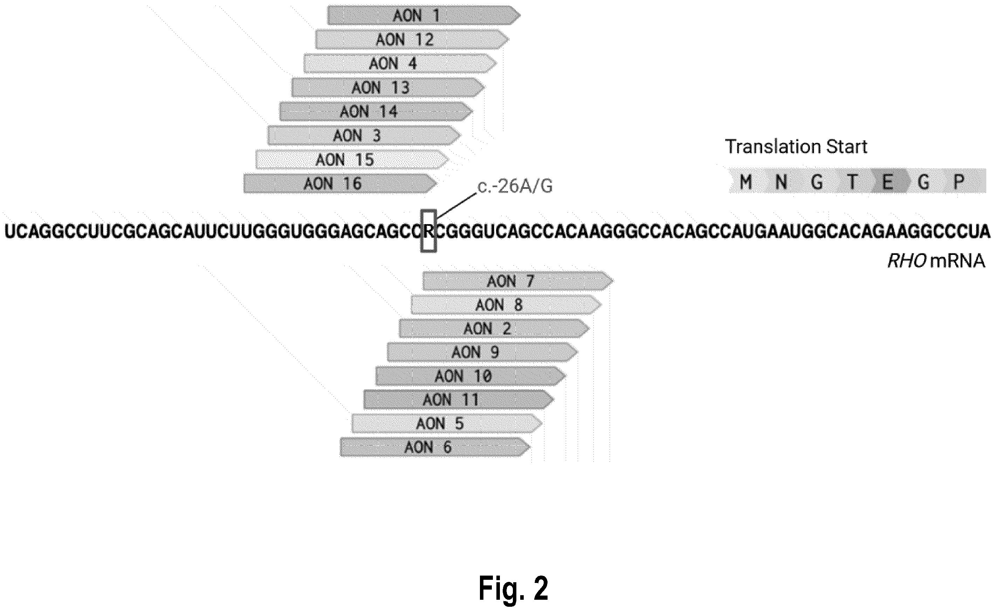

- Fig. 2:: Target sequence of the screened AONs in the *RHO* mRNA. Schematic depiction of the target region of 16 tested gapmer AONs in the 5' untranslated region (UTR) of the *RHO* mRNA. At position +1 (AUG), the start of the amino acid sequence is shown.

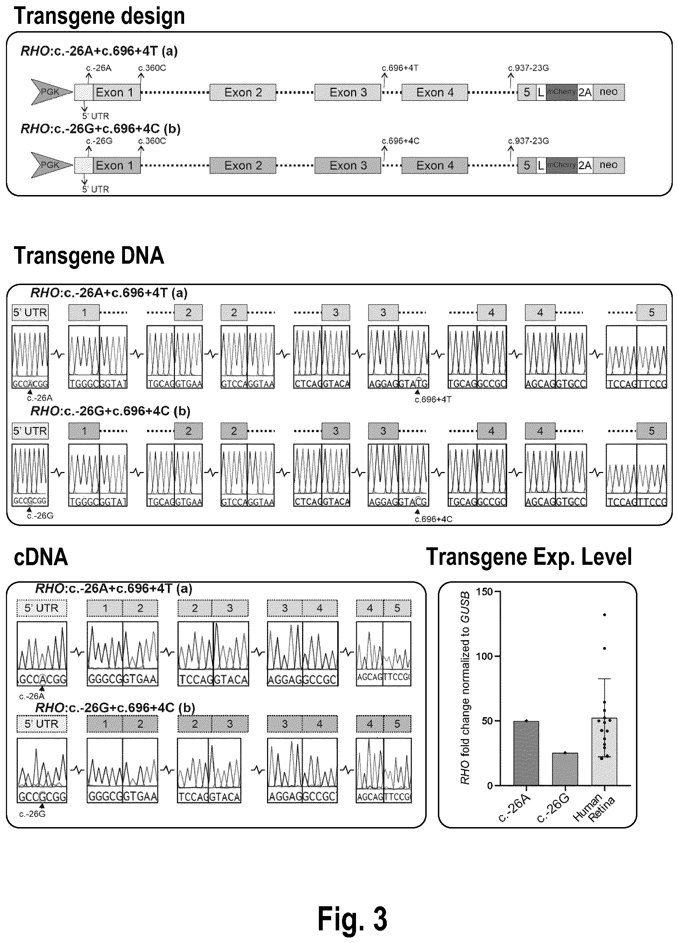

- Fig. 3:: Validation of the c.-26A/G *RHO-*expressing reporter HEK293T cell lines at the DNA and RNA levels. The top panel represents the schematic design of the two *RHO* transgene stably integrated in the reporter cell lines. The middle panel shows the sequencing of the integrated transgene at the genomic DNA level. The bottom left panel represents cDNA validation, showing cor- rect splicing of the *RHO* transgene. The bottom right panel shows the level of expression of the *RHO* transgenes normalized to GUSB in comparison to the *RHO* expression level in the whole human retina.

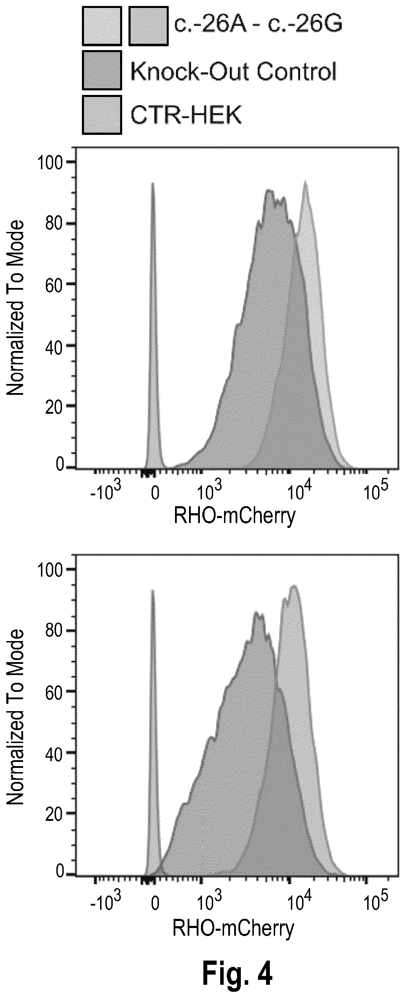

- Fig. 4:: Validation of the c.-26A/G *RHO*-expressing reporter HEK293T cell lines at the protein level. Flow cytometry results of the transgene expression as measured by mCherry expression for the c.-26A (green) and c.-26G (orange) reporter cell lines. Deletion of exon 2 through plasmid-delivered 2gRNA-CRISPR/Cas9 was used to confirm the response of the reporter cell line to the transgene expression knock-out (Knock-Out Control). CTR-HEK = Control HEK293T non expressing for the transgene.

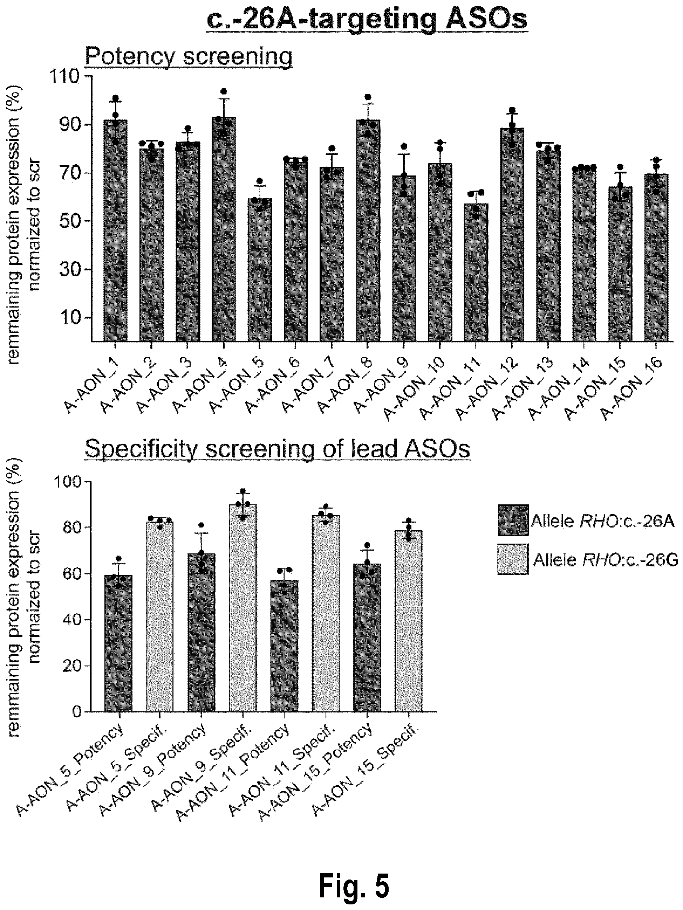

- Fig. 5:: Screening of c.-26A-targeting gapmer AONs in the *RHO*-expressing reporter HEK293T cell lines to assess potency and specificity. The results are represented as the mean ± standard deviation of 4 independent experimental replicates and express the value of the protein knock-down level detected after treatment as per flow cytometry results. Potency was assessed in the *RHO*:c.-26A-expressing reporter cell line (top panel). Specificity for the 4 most potent gapmer AONs was tested in the *RHO*:c.-26G-expressing reporter cell line (bottom panel).

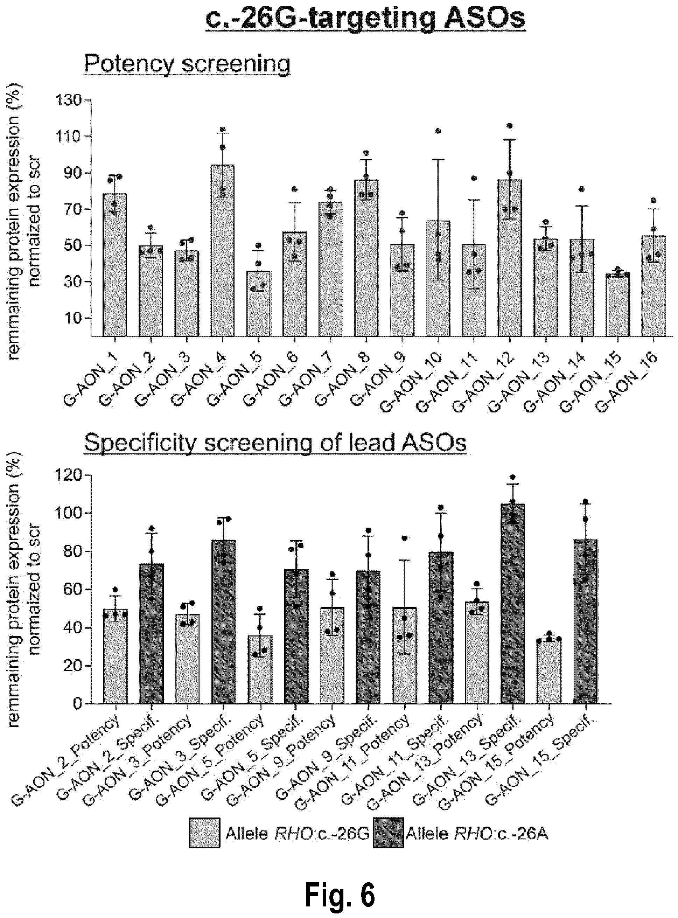

- Fig. 6:: Screening of c.-26G-targeting gapmer AONs in the *RHO*-expressing reporter HEK293T cell lines to assess potency and specificity. The results are represented as the mean ± standard deviation of 4 independent experimental replicates and express the value of the protein knock-down level detected after treatment as per flow cytometry results. Potency was assessed in the *RHO*:c.-26G-expressing reporter cell line (top panel). Specificity (Specif.) for the 4 most potent gapmer AONs was tested in the *RHO*:c.-26A-expressing reporter cell line (bottom panel).

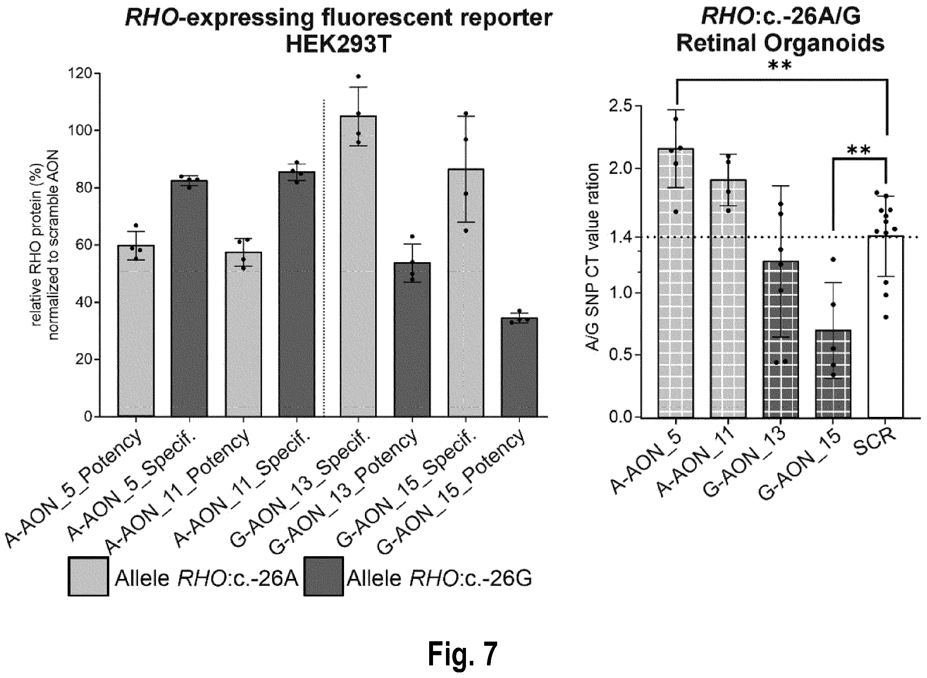

- Fig. 7:: Validation of two lead gapmer AONs in *RHO*:c.-26A/G retinal organoids. As a reference, the potency and specificity results obtained in the RHO-express- ing reporter cell lines are reported in the left panel. Retinal organoids were treated with 5uM of naked gapmer AONs for 10 days. Results are repre- sented as the mean ± standard deviation of the ration of the CT values ob- tained by allele-specific TaqMan assay.

### EXAMPLES

### 1. gAON-based strategy for mutation-independent knock-down of RHO

The inventors propose to explore and further develop a novel therapeutic strategy which selectively lowers the amount of the mutant RHO protein in a mutation-independent approach. The approach utilizes gapmer antisense oligonucleotides (gAONs), chemically modified single-stranded antisense oligonucleotides with a DNA core and RNA wings which binds to target mRNA molecules and induced RNAse H-based cleavage and destruction of the target molecules; see Mangos et al. (2003), "Efficient RNase H-directed cleavage of RNA promoted by antisense DNA or 2'F-ANA constructs containing acyclic nucleotide inserts", Journal of the American Chemical Society 125(3), pp. 654-661.

Enabling to address a large subcohort of patients, according to the invention, the therapeutic strategy for treatment of *RHO*-associated autosomal dominant Retinitis Pigmentosa does not target a specific disease-causing variant but rather harnesses the abundance of a frequent SNP at cDNA sequence position -26 (c.-26) in the *RHO* 5' untranslated region (UTR). This polymorphism is common in both patients and healthy individuals and exists independently of the respective mutation. While the majority of alleles show an A at this position, numerous individuals exhibit alleles with c.-26G. According to the gnomAD database, in around 25% of sequenced genomes, this SNP is found in a heterozygous state, meaning one *RHO* allele exhibits c.-26A while the other presents c.-26G (https://gnomad.broadinstitute.org).

In the patient cohort examined by the inventors, across 110 non-related *RHO*-associated autosomal dominant retinitis pigmentosa patients, 31 (28%) were geno-typed to harbor the c.-26A/G in heterozygosity. Phasing revealed that *RHO* mutations were in 18/31 patients (58%) in cis with c.-26A and in 13/31 patients (42%) in cis with c.-26G. This heterozygosity is the basis and prerequisite for the mutation-independent antisense oligonucleotide approach according to the invention. Patients that exhibit a pathogenic, dominant *RHO* variant and are heterozygous for this SNP could potentially be treated with an effective gAON that selectively targets the SNP, being either A or G, in cis with the respective pathogenic variant. This should result in reduced expression of the defective protein and, depending on the variant, alleviate the patient's symptomatic. The gAON-based strategy for mutation-independent knock-down of *RHO* underlying the invention is illustrated in Fig. 1.

### 2. Target sequence of the screened AONs in the RHO mRNA

In total 32 gapmer AONs were tested regarding their knock-down potency: 16 gapmer AONs targeting the SNP c.-26A and 16 gapmer targeting the SNP c.-26G. Their target sequences are depicted in Fig. 2.

### 3. Gapmer-AONs

All used gapmer-AONs were composed of 16 nucleotides and a (PS) backbone. The central DNA part consisted of 10 DNA bases encompassed by three 2'-*O-*methoxyethyl- (2'M*O*E) oligoribonucleotides on each side. The gapmers targeting *RHO*:c.-26A were supplied by ProQR Therapeutics NV (Leiden, Netherlands) and the ones for *RHO*:c.-26G were purchased from Eurogentec (Seraing, Belgium).

### 4. HEK293T RHOc.-26A/G-mCherry-ble

For potency and specificity screenings of antisense oligonucleotides, two reporter cell lines HEK293T *RHO*:c.-26A-mCherry-ble and *RHO*:c.-26G-mCherry-ble were used. These transgenic derivate of HEK293T stably expresses a transgene composed of the whole *RHO* coding genomic DNA (including intervening introns), harboring either *RHO*:c.-26A or *RHO*:c.-26G fused to mCherry protein and ble (Bleomycin resistance protein) as selection marker.

### 5. Validation of the c.-26A/G RHO-expressing reporter HEK293T cell lines at the DNA and RNA levels

The inventors validated the c.-26A/G *RHO*-expressing reporter HEK293T cell lines at the DNA and RNA levels. The results of these experiments are shown in Fig. 3. The top panel represents the schematic design of the two *RHO* transgene stably integrated in the reporter cell lines. The middle panel shows the sequencing of the integrated transgene at the genomic DNA level. The bottom left panel represents cDNA validation, showing correct splicing of the *RHO* transgene. The bottom right panel shows the level of expression of the *RHO* transgenes normalized to GUSB in comparison to the *RHO* expression level in the whole human retina.

As can be seen in Fig. 3, the integrated *RHO*-mCherry transgene in the established RHO-expressing reporter cell lines demonstrates the presence of either c.-26A/G SNP in the 5'-UTR and a second common SNP (c.696+4C/T) in intron 3. The presence of the c.-26A/G SNP is further confirmed in the transgene transcript, as validated by cDNA analysis. Relative quantification of transgene transcripts by qRT-PCR revealed fold-change expression levels of 49.7 and 25.5 compared to *GUSB* for the c.-26A- and c.-26G-expressing RHO reporter cell lines, respectively. In comparison, RNA-seq data from 19 human whole retinas were used to estimate the endogenous expression level of *RHO,* which showed a 43.7±31.8 fold-change compared to *GUSB.*

### 6. Validation of the c.-26A/G RHO-expressing reporter HEK293T cell lines at the protein level

The inventors then validated the c.-26A/G *RHO*-expressing reporter HEK293T cell lines at the protein level. The results are depicted in Fig. 4. Flow cytometry results of the transgene expression as measured by mCherry expression for the c.-26A (green) and c.-26G (orange) reporter cell lines. Deletion of exon 2 through plasmid-delivered 2gRNA-CRISPR/Cas9 was used to confirm the response of the reporter cell line to the transgene expression knock-out (Knock-Out Control). CTR-HEK = Control HEK293T non expressing for the transgene.

What is shown in Fig. 4, is histograms from flow cytometry analysis of the RHO-expressing reporter cell lines. The x-axis represents the logarithmic fluorescence intensity for mCherry, while the y-axis displays the normalized mode of the registered events. The top histogram illustrates the c.-26A-expressing reporter cell line population, showing a median fluorescence intensity of 22078. Upon treatment with 2g RNA-CRISPR/Cas9, the median fluorescence intensity was reduced to 5900, indicating a successful transgene knock-out response. Similarly, the bottom histogram depicts the c.-26G-expressing reporter cell line population, with a median fluorescence intensity of 12467, which also decreased to 3561 following 2gRNA-CRISPR/Cas9 treatment, confirming the knock-out effect. As a control, HEK293T cells lacking the transgene displayed a median autofluorescence intensity of 94 in the mCherry channel.

### 7. Delivery of gapmer-AONs by Lipofectamine 3000 in HEK293T RHOc.-26A/G-mCherry-ble

For delivery of gapmer-AONs by means of lipofection, 400,000 of the respective HEK293T reporter cells were seeded in 1 ml of DMEM + 10% FBS + respective selection antibiotic. Before transfection the next day, the medium was replaced by 900 µl of DMEM without FBS and antimicrobials. 49,4 µl of OptiMEM were incubated with the Lipofectamine^{®} 3000 reagent for 5 min and then combined with a mixture of 3 µl gapmer-AON and 47 µl Opti-MEM. The final concentration of the gapmer-AON in the medium was 50nM. After an incubation time of 5 min, this mixture was added to the cells in a dropwise manner. Three days of incubation at 37°C and 5% CO₂ later, the cells were harvested and analyzed by flow cytometry as described in the following paragraph.

### 8. Flow cytometry

To evaluate expression of *RHO* transgenes by fluorescence of the fluorescent protein (mCherry), flow cytometry was employed. HEK293T cells were harvested and the cells finally resuspended in PBS at a final concentration ranging from 250,000 to 2 million cells/ml in a 5 ml FACS tube. Cells that have undergone starvation, were additionally filtered through a 100 µM filter to avoid cell clumps. As a negative control to set the acquisition parameters, non-transfected cells were also included for each experiment. The tubes were placed on ice for transport and the cells were analyzed on a BD FACS Fortessa at the Flow Cytometry Core Facility Berg of the Medical Faculty of the University of Tübingen.

To differentiate the cell population of interest while excluding debris, cells were first analyzed by plotting forward (FSC) and side scatters (SSC) signals, allowing the identification and gating of only living cells. To discriminate between single cells (singlets) from cell clumps (duplets), the area of the FSC signal (FSC-A) was plotted against the height of the same signal (FSC- H). A 561 nm laser for detection of mCherry.

### 9. Screening of gapmer AONs in the RHO:c.-26A/G-expressing reporter cell lines using flow cytometry

Differences in mCherry signal and thus *RHO*-mCherry protein level were analyzed using FlowJoTM (BD Biosciences). To normalize relative protein knock-down, each experiment included cells treated with a scramble gAON, which had the same length as the screened gAONs but exhibited a random sequence and thus does not lead to any knock-down. The median mCherry level of the respective AON-treated cell sample was divided by the median mCherry level of cells treated with the scramble AON and multiplied by 100 to obtain a relative percentage.

The results of the screening of the 16 *RHO*:c.-26A-targetting and 16 *RHO*:c.-26G-targetting gapmer AONs in the reporter cell lines are shown in Figs. 5 (c.-26A-targeting AONs) and 6 (c.26G-targeting AONs). The results are represented as the mean ± standard deviation of 4 independent experimental replicates and express the value of the remaining protein expression (%) normalized to scramble after treatment as per flow cytometry results. Potency was assessed in the *RHO*:c.-26A-expressing (Fig. 5) or *RHO*:c.-26G-expressing (Fig. 6) reporter cell line (top panel). Specificity for the 4 most potent gapmer AONs was tested in the c.-26G-expressing or c.-26A-expressing reporter cell line, respectively (bottom panel).

As can be seen for the c.-26A-targeting gAONs in Fig.5 top panel, the treatment of the c.-26A-expressing reporter cell line with the 16 *RHO*:c.-26A-targetting gAONs yielded to a remaining protein expression (%) normalized to scramble in the range of 57.45±4.86% to 93.10±7.52% or a knock-down level of 42.55±4.86% to 6.90±7.52%, respectively. In Fig.5 bottom panel, specificity analysis is shown for candidates A-AON_5, A-AON_9, A-AON_11, and A-AON_15 performed in the c.-26G-expressing reporter cell line. The remaining protein expression (%) normalized to scramble ranged between 78.75±3.50% and 90.0±4.9% or a knock-down level of 21.25±3.50% to 10.0±4.9%, respectively. This indicates that lead c.-26A-targeting gAONs show potency for the target *RHO* allele and specificity for the counter allele.

For the c.-26G-targeting gAONs in Fig.6 top panel, the treatment of the c.-26G-expressing reporter cell line with the 16 *RHO*:c.-26G-targetting gAONs yielded to a remaining protein expression (%) normalized to scramble in the range of 34.50±1.73% to 94.25±17.56% or a knock-down level of 65.50±1.73% to 5.75±17.56%, respectively. In Fig.6 bottom panel, specificity analysis is shown for candidates G-AON_2, G-AON_3, G-AON_5, G-AON_9, G-AON_11, G-AON_13, and G-AON_15 performed in the c.-26A-expressing reporter cell line. The remaining protein expression (%) normalized to scramble ranged between 70.00±17.94% and 105.00±10.23% or a knock-down level of 30.00±17.94% to - 5.00±10.23%, respectively. This indicates that lead c.-26G-targeting gAONs show potency for the target *RHO* allele and specificity for the counter allele.

### 10. Validation of lead gapmer AONs in RHO:c.-26A/G retinal organoids

To evaluate whether gapmer AONs can be effectively delivered gymnotically (naked delivery) in an advanced cellular model, such as mature retinal organoids, and to assess their ability to mediate allele-specific knockdown of endogenous *RHO* expression, 5 µM of the two lead gapmer AONs targeting c.-26A and two targeting c.-26G were added to retinal organoids. After ten days, total mRNA was extracted, reverse-transcribed into cDNA, and the ratio of c.-26A- to c.-26G-containing transcripts was quantified using a highly specific TaqMan assay. The results are depicted in Fig. 7. As a reference, the potency and specificity results obtained in the RHO-expressing reporter cell lines are reported in the left panel. Retinal Organoids were treated with 5 µM of naked gapmer AONs for 10 days. Results are represented as the mean ± standard deviation of the ration of the CT values obtained by allele-specific TaqMan assay.

The potencies and specificities of the gAONs according to the invention in *RHO*-expressing reporter cells and retinal organoids are summarized in Table 1.

It can be seen an increase in the c.-26A/c.-26G ratio for the c.-26A-targeting gapmer AONs and a decrease for the c.-26G-targeting gapmer AONs. This indicates that the molecules can effectively diffuse into retinal organoids and mediate allele-specific knock-down of endogenous *RHO* expression.

### Potency in RHO-expressing reporter cells

| | **A-AON_1** | **A-AON_2** | **A-AON_3** | **A-AON_4** | **A-AON_5** | **A-AON_6** | **A-AON_7** | **A-AON_8** | **A-AON_9** | **A-AON_10** | **A-AON_11** | **A-AON_12** | **A-AON_13** | **A-AON_14** | **A-AON_15** | **A-AON_16** |
|---|---|---|---|---|---|---|---|---|---|---|---|---|---|---|---|---|
| **Number of values** | 4 | 4 | 4 | 4 | 4 | 4 | 4 | 4 | 4 | 4 | 4 | 4 | 4 | 4 | 4 | 4 |
| | | | | | | | | | | | | | | | | |
| **Mean** | 91,95 | 80,2 | 83 | 93,1 | 59,5 | 74,53 | 72,45 | 91,98 | 68,93 | 74,13 | 57,45 | 88,68 | 79,2 | 71,98 | 64,28 | 69,63 |
| **Std. Deviation** | 7,576 | 3,18 | 3,64 | 7,524 | 4,998 | 1,628 | 5,322 | 6,641 | 8,686 | 8,361 | 4,862 | 5,92 | 3,087 | 0,3594 | 5,935 | 5,743 |
| **Std. Error of Mean** | 3,788 | 1,59 | 1,82 | 3,762 | 2,499 | 0,8138 | 2,661 | 3,321 | 4,343 | 4,181 | 2,431 | 2,96 | 1,543 | 0,1797 | 2,967 | 2,872 |
| | | | | | | | | | | | | | | | | |

| | **G-AON_1** | **G-AON_2** | **G-AON_3** | **G-AON_4** | **G-AON_5** | **G-AON_6** | **G-AON_7** | **G-AON_8** | **G-AON_9** | **G-AON_10** | **G-AON_11** | **G-AON_12** | **G-AON_13** | **G-AON_14** | **G-AON_15** | **G-AON_16** |
|---|---|---|---|---|---|---|---|---|---|---|---|---|---|---|---|---|
| **Number of values** | 4 | 4 | 4 | 4 | 4 | 4 | 4 | 4 | 4 | 4 | 4 | 4 | 4 | 4 | 4 | 4 |
| | | | | | | | | | | | | | | | | |
| **Mean** | 78,75 | 50 | 47,25 | 94,25 | 36 | 57,5 | 74 | 86,25 | 50,75 | 64 | 50,75 | 86,5 | 53,75 | 53,5 | 34,5 | 55,5 |
| **Std. Deviation** | 9,777 | 6,683 | 5,56 | 17,56 | 11,2 | 16,18 | 6,481 | 10,9 | 14,73 | 33,22 | 24,58 | 21,81 | 6,652 | 18,36 | 1,732 | 14,82 |
| **Std. Error of Mean** | 4,888 | 3,342 | 2,78 | 8,779 | 5,598 | 8,088 | 3,24 | 5,452 | 7,364 | 16,61 | 12,29 | 10,9 | 3,326 | 9,179 | 0,866 | 7,411 |

### Specificity in RHO-expressing reporter cells

| | **A-AON_5_ Specificity** | **A-AON_9_ Specificity** | **A-AON_11_Specificity** | **A-AON_15 Specificity** |
|---|---|---|---|---|
| **Number of values** | 4 | 4 | 4 | 4 |
| | | | | |
| **Mean** | 82,5 | 90 | 85,5 | 78,75 |
| **Std. Deviation** | 1,732 | 4,899 | 2,887 | 3,5 |
| **Std. Error of Mean** | 0,866 | 2,449 | 1,443 | 1,75 |

| | **G-AON_2-Specificity** | **G-AON_3-Specificity** | **G-AON_5-Specificity** | **G-AON_9-Specificity** | **G-AON_11-Specificity** | **G-AON_13-Specificity** | **G-AON_15-Specificity** |
|---|---|---|---|---|---|---|---|
| **Number of values** | 4 | 4 | 4 | 4 | 4 | 4 | 4 |
| | | | | | | | |
| **Mean** | 73,5 | 86 | 70,75 | 70 | 79,75 | 105 | 86,5 |
| **Std. Deviation** | 16,01 | 11,69 | 14,75 | 17,94 | 20,27 | 10,23 | 18,48 |
| **Std. Error of Mean** | 8,005 | 5,845 | 7,375 | 8,972 | 10,14 | 5,115 | 9,242 |

### c.-26A/G Retinal Organoids

**Tab. 1: Potency and specificity of gAONs in RHO-expressing reporter cells and retinal organoids**

| | **A-AON5** | **A-AON11** | **G-AON13** | **G-AON15** | **SCR** |
|---|---|---|---|---|---|
| **Number of values** | 6 | 4 | 8 | 5 | 12 |
| | | | | | |
| **Mean** | 2,151 | 1,901 | 1,248 | 0,6959 | 1,451 |
| **Std. Deviation** | 0,3109 | 0,2064 | 0,6051 | 0,3842 | 0,3208 |
| **Std. Error of Mean** | 0,1269 | 0,1032 | 0,2139 | 0,1718 | 0,09261 |

### 11. Conclusion

The invention provides for the first time a mutation-independent strategy that can successfully treat a significant proportion of retinitis pigmentosa (RP) patients. To this end, the invention uses so-called gapmer antisense oligonucleotide (gAONs), which specifically and selectively hybridize to a single nucleotide polymorphism (SNP) in the RHO mRNA, upstream of the coding sequence. In patients heterozygous for the SNP, only the disease-causing RHO allele is degraded via an RNase H-dependent mechanism, resulting in reduced expression of mutant RHO protein. The predominance of healthy RHO protein leads to a significant improvement in the clinical picture or even a complete cure of the patient. 
SEQUENCES

Target

DNA: TGGGTGGGAGCAGCCRCGGGTCAGCCACAAG (R = A or G) (SEQ ID NO: 1)
RNA: UGGGUGGGAGCAGCCRCGGGUCAGCCACAAG (R = A or G) (SEQ ID NO: 2)

gAONs

targeting c-26A

A-AON_5:
Full: <G*C*T*>G*A*C*C*C*G*T*G*G*C*<T*G*C> (SEQ ID NO: 3)
Gap: G*A*C*C*C*G*T*G*G*C* (SEQ ID NO: 33)

At the sequence segment in angle brackets (<...>), a uracil (U) can be pro-
vided instead of a thymine (T).

A-AON_11:

Full: <G*G*C*>T*G*A*C*C*C*G*T*G*G*<C*T*G> (SEQ ID NO: 4)
Gap: T*G*A*C*C*C*G*T*G*G* (SEQ ID NO: 34)

At the sequence segment in angle brackets (<...>), a uracil (U) can be pro-
vided instead of a thymine (T).

Targeting c.26G

G-AON_13:
Full: <C*C*C*>G*C*G*G*C*T*G*C*T*C*<C*C*A> (SEQ ID NO: 5)
Gap: G*C*G*G*C*T*G*C*T*C* (SEQ ID NO: 35)

At the sequence segment in angle brackets (<...>), a uracil (U) can be pro-
vided instead of a thymine (T).

G-AON_15:
Full: <G*C*G*>G*C*T*G*C*T*C*C*C*A*<C*C*C> (SEQ ID NO: 6)
Gap: G*C*T*G*C*T*C*C*C*A* (SEQ ID NO: 36)

At the sequence segment in angle brackets (<...>), a uracil (U) can be pro-
vided instead of a thymine (T).

Legend:

* = Phosphorothioates
2'-O-MOE (2'-O-methoxyethyl) RNA = <NNN>

The 10 nucleotides in the middle (the gap part) are DNA whereas the 3bp ei-
ther end are 2'-O-MOE RNA
 
A-AON_5:      G   C   T   G   A   C   C   C   G   T   G   G   C   T   G   C
A-AON_11: G   G   C   T   G   A   C   C   C   G   T   G   G   C   T   G
G-AON_13:                         C   C   C   G   C   G   G   C   T   G   C   T   C   C   C   A
G-AON_15:                                     G   C   G   G   C   T   G   C   T   C   C   C   A   C   C   C

Cons.:    X1  X2  X3  X4  X5  X6  X7  X8  X9  G   X10 G   G   C   T   G   X11 X12 X13 X14 X15 X16 X17 X18 X19

with

X1   = absent or G
X2   = absent or G
X3   = absent of C
X4   = absent or T/U
X5   = absent or G
X6   = absent or A
X7   = absent or C
X8   = absent or C
X9   = absent or C
X10  = C or T/U
X11  = absent or C
X12  = absent or T/U
X13  = absent or C
X14  = absent or C
X15  = absent or C
X16  = absent or A
X17  = absent or C
X18  = absent or C
X19  = absent or C

(SEQ ID NO: 37)

A-AON_3     <C*G*T*>G*G*C*T*G*C*T*C*C*C*<A*C*C> (SEQ ID NO: 7)
A-AON_4     <A*C*C*>C*G*T*G*G*C*T*G*C*T*<C*C*C> (SEQ ID NO: 8)
A-AON_5     <G*C*T*>G*A*C*C*C*G*T*G*G*C*<T*G*C> (SEQ ID NO: 3)
A-AON_6     <C*T*G*>A*C*C*C*G*T*G*G*C*T*<G*C*T> (SEQ ID NO: 9)
A-AON_7     <C*T*T*>G*T*G*G*C*T*G*A*C*C*<C*G*T> (SEQ ID NO: 10)
A-AON_8     <T*T*G*>T*G*G*C*T*G*A*C*C*C*<G*T*G> (SEQ ID NO: 11)
A-AON_9     <G*T*G*>G*C*T*G*A*C*C*C*G*T*<G*G*C> (SEQ ID NO: 12)
A-AON_10    <T*G*G*>C*T*G*A*C*C*C*G*T*G*<G*C*T> (SEQ ID NO: 13)
A-AON_11    <G*G*C*>T*G*A*C*C*C*G*T*G*G*<C*T*G> (SEQ ID NO: 4)
G-AON_12    <G*A*C*>C*C*G*T*G*G*C*T*G*C*<T*C*C> (SEQ ID NO: 14)
G-AON_13    <C*C*C*>G*T*G*G*C*T*G*C*T*C*<C*C*A> (SEQ ID NO: 15)
G-AON_14    <C*C*G*>T*G*G*C*T*G*C*T*C*C*<C*A*C> (SEQ ID NO: 16)
G-AON_15    <G*T*G*>G*C*T*G*C*T*C*C*C*A*<C*C*C> (SEQ ID NO: 17)
G-AON_16    <T*G*G*>C*T*G*C*T*C*C*C*A*C*<C*C*A> (SEQ ID NO: 18)

G-AON_1     <T*G*A*>C*C*C*G*C*G*G*C*T*G*<C*T*C> (SEQ ID NO: 19)
G-AON_2     <T*G*T*>G*G*C*T*G*A*C*C*C*G*<C*G*G> (SEQ ID NO: 20)
G-AON_3     <C*G*C*>G*G*C*T*G*C*T*C*C*C*<A*C*C> (SEQ ID NO: 21)
G-AON_4     <A*C*C*>C*G*C*G*G*C*T*G*C*T*<C*C*C> (SEQ ID NO: 22)
G-AON_5     <G*C*T*>G*A*C*C*C*G*C*G*G*C*<T*G*C> (SEQ ID NO: 23)
G-AON_6     <C*T*G*>A*C*C*C*G*C*G*G*C*T*<G*C*T> (SEQ ID NO: 24)
G-AON_7     <C*T*T*>G*T*G*G*C*T*G*A*C*C*<C*G*C> (SEQ ID NO: 25)
G-AON_8     <T*T*G*>T*G*G*C*T*G*A*C*C*C*<G*C*G> (SEQ ID NO: 26)
 
G-AON_9     <G*T*G*>G*C*T*G*A*C*C*G*C*<G*G*C> (SEQ ID NO: 27)
G-AON_10    <T*G*G*>C*T*G*A*C*C*G*C*G*<G*C*T> (SEQ ID NO: 28)
G-AON_11    <G*G*C*>T*G*A*C*C*G*C*G*G*<C*T*G> (SEQ ID NO: 29)
G-AON_12    <G*A*C*>C*C*G*C*G*G*C*T*G*C*<T*C*C> (SEQ ID NO: 30)
G-AON_13    <C*C*C*>G*C*G*G*C*T*G*C*T*C*<C*C*A> (SEQ ID NO: 5)
G-AON_14    <C*C*G*>C*G*G*C*T*G*C*T*C*C*<C*A*C> (SEQ ID NO: 31)
G-AON_15    <G*C*G*>G*C*T*G*C*T*C*C*C*A*<C*C*C> (SEQ ID NO: 6)
G-AON_16    <C*G*G*>C*T*G*C*T*C*C*C*A*C*<C*C*A> (SEQ ID NO: 32)

At the sequence segment in angle brackets (<...>), a uracil (U) can be pro-
vided instead of a thymine (T).
 In case of discrepancies between the above sequences and those from the sequence listing, the above sequences take precedence

## Claims

1. A gapmer antisense oligonucleotide (gAON) that specifically binds to and/or is complementary to a polynucleotide comprising the nucleotide sequence of SEQ ID NO: 1 and/or SEQ ID NO: 2.

2. The gAON of claim 1 configured to mediate degradation by RNase of an mRNA and/or pre-mRNA encoding the human *RHO* gene, preferably the mutated human *RHO* gene, or fragments thereof.

3. The gAON of claim 1 or 2, comprising a DNA sequence ("gap") of approx. 6 to approx. 20 nucleotides, preferably approx. 8 to approx. 15 nucleotides, particularly preferably approx. 10 nucleotides.

4. The gAON of claim 3, wherein the gap comprises a nucleotide sequence selected from the group consisting of SEQ ID NO: 33 - 36.

5. The gAON of any of the preceding claims, comprising an additional RNA sequence ("wing"), at its 3' and/or 5' terminus, of approx. 2 to approx. 10 nucleotides, preferably approx. 2 to approx. 5 nucleotides, particularly preferably approx. 3 nucleotides.

6. The gAON of claim 5, wherein the wing comprises modified bases.

7. The gAON of claim 6, wherein the modification is selected from the group consisting of 2'-*O*-methylation (2'-M*O*E), locked nucleic acid (LNA), and 2 fluoro (2'-F) modification.

8. The gAON of any of the preceding claims 1, comprising the following consensus nucleotide sequence:

X1-X2-X3-X4-X5-X6-X7-X8-X9-G-X10-G-G-C-T-G-X11-X12-X13-X14-X15-X16-X17-X18-X19, with

X1 = absent or G

X2 = absent or G
X3 = absent of C
X4 = absent or T/U
X5 = absent or G
X6 = absent or A
X7 = absent or C
X8 = absent or C
X9 = absent or C
X10 = C or T/U
X11 = absent or C
X12 = absent or T/U
X13 = absent or C
X14 = absent or C
X15 = absent or C
X16 = absent or A
X17 = absent or C
X18 = absent or C
X19 = absent or C (SEQ ID NO: 37).

9. The gAON of any of the preceding claims, **characterized in that** it comprises a nucleotide sequence selected from the group consisting of SEQ ID NO: 3 - 6.

10. The gAON of any of claims 1-7, wherein it comprises a nucleotide sequence selected from the group consisting of SEQ ID NO: 3 - 32.

11. A pharmaceutical composition comprising the gAON of any of claims 1-10 and a pharmaceutically acceptable excipient.

12. The pharmaceutical composition of claim 11 configured for intravitreal administration.

13. The gAON of any of claims 1-10 and/or the pharmaceutical composition of claim 11 or 12 for use as a medicament, preferably for use in the treatment of retinitis pigmentosa (RP), preferably autosomal dominant RP.

14. A method for modulating the expression of the human *RHO* gene, preferably the mutated human RHO gene, in a biological cell, preferably a rod cell, said method comprising contacting said biological cell with the gAON of any of claims 1-10 and/or the pharmaceutical composition of claim 11 or 12.

15. A method for the prophylaxis and/or treatment of a living being against and/or suffering from RP, preferably autosomal dominant RP, comprising the administration to said living being of the gAON of any of claims 1-10 and/or the pharmaceutical composition of claim 11 or 12.
